# EUROPÄISCHE PATENTSCHRIFT

(11) **EP 2 150 351 B1**
(45) Veröffentlichungstag und Bekanntmachung des Hinweises auf die Patenterteilung: **11.05.2011**
(21) Anmeldenummer: 08760504.4
(22) Anmeldetag: 04.06.2008
(51) Int. Cl.: B04B 5/04

(54) **EINSATZ UND ZENTRIFUGE MIT EINSATZ**
INSERT AND CENTRIFUGE COMPRISING AN INSERT
PIÈCE RAPPORTÉE ET CENTRIFUGEUSE À PIÈCE RAPPORTÉE

(30) Priorität: 05.06.2007 DE 102007000310
(43) Veröffentlichungstag der Anmeldung: 10.02.2010
(73) Patentinhaber: Terumo Europe NV, 3001 Leuven (BE); Andreas Hettich GmbH&Co. KG, 78532 Tuttlingen (DE)
(72) Erfinder: EBERLE, Klaus-Günter, 78532 Tuttlingen (DE); BISET, Roland, B-3001 Leuven (BE)
(74) Vertreter: TBK-Patent
(86) Internationale Anmeldenummer: PCT/EP2008/056923
(87) Internationale Veröffentlichungsnummer: WO 2008/148808

(56) Entgegenhaltungen:
- EP-A- 0 499 891
- EP-A- 0 616 816
- EP-B- 1 351 772
- US-A- 5 543 062

## Beschreibung

### Technisches Gebiet

Die Erfindung betrifft einen Einsatz, der insbesondere zur Verwendung in einer Zentrifuge vorgesehen ist, um Blut in seine Bestandteile aufzutrennen, und eine Zentrifuge mit einem solchen Einsatz.

### Stand der Technik

In der Transfusionsmedizin hat sich seit dem Beginn der neunziger Jahre die so genannte Blutkomponententherapie durchgesetzt. Dies bedeutet, dass einem Patienten anstelle einer Vollblutkonserve lediglich diejenigen Blutbestandteile verabreicht werden, die der einzelne Patient benötigt. Durch diese getrennte Verabreichung der einzelnen Blutbestandteile ist es möglich, mit einer einzigen Blutkonserve durchschnittlich 1,8 Patienten optimal zu helfen.

Die wesentlichen Blutbestandteile sind
die roten Blutkörperchen im so genannten Erythrozytenkonzentrat, die zur Aufrechterhaltung der Sauerstoffversorgung nach schweren Blutverlusten transfundiert werden,
die Blutplättchen im Thrombozytenkonzentrat, die bei Gerinnungsstörungen (Bluterkrankheit) verabreicht werden, und
das Blutplasma, das bei Gerinnungsstörungen und Volumendefiziten verabreicht wird. Abgesehen davon ist Blutplasma ein wesentlicher Grundbestandteil zur Herstellung vieler Medikamente.

Das Auftrennen der einzelnen Blutbestandteile, das als Zellgewinnung bezeichnet wird, erfolgt bekannt durch ein Behandeln des Bluts in einer Zentrifuge. Durch das Zentrifugieren werden die einzelnen Blutbestandteile voneinander getrennt und können dann einzeln in entsprechende Behältnisse abgefüllt und verwendet werden.

Beispielsweise ist aus der EP 1 351 772 B1 eine derartige Zentrifuge bekannt. Gemäß diesem Stand der Technik sind in einem Rotor einer Zentrifuge eine Vielzahl von Einsätzen um eine Nabe angeordnet. Die Einsätze sind dabei fest in dem Rotor gehalten, sodaß die Blutbeutel stehend zentrifugiert werden. Die Einsätze weisen in Ihrem Inneren

Aufnahmen für einen Vollblut enthaltenden Blutbeutel und für Produktbeutel auf, in denen das Plasma bzw. das Erythrozytenkonzentrat gesammelt wird. Um zu verhindern, dass es nach erfolgter Trennung der einzelnen Bestandteile zu einem Nachfließen und abermaligen Vermischen der Produkte kommt, sind in dem Einsatz verschiedene Klemmeinrichtungen vorgesehen, mit denen die einzelnen Schläuche abgeklemmt werden können. Zum Entnehmen der Beutel aus dem Einsatz nach erfolgter Trennung, müssen die einzelne Anschlusschläuche der Beutel durch geeignete Maßnahmen verschlossen werden. Erst danach können die Klemmen des Einsatzes geöffnet, die Beutel entnommen und der Einsatz zur Aufnahme eines neuen Beutelsatzes vorbereitet werden.

Nach der Trennung und dem Abfüllen des Plasmas bzw. der roten Blutkörperchen verbleibt ein als "buffy coat" bezeichnetes Gemisch in den Blutbeuteln. Dieses "buffy coat" besteht im Wesentlichen aus Blutplättchen sowie weißen und roten Blutkörperchen. Zur Gewinnung der Blutplättchen aus diesem "buffy coat" wird dieses mit einer Additivlösung verdünnt und dieses verdünnte "buffy coat" durch Zentrifugieren abermals in seine Bestandteile aufgetrennt.

Aus der WO 03/089027 ist ein System und Verfahren zu diesem Zweck bekannt. Diese Druckschrift offenbart eine Zentrifuge, in deren einzelner Kammer ein ringförmiger Beutel mit einem Gemisch aus "buffy coat" und Additivlösung eingelegt werden. Die Blutbestandteile werden dann durch einen Zentrifugiervorgang aufgetrennt und die getrennten Bestandteile durch eine Schlauchleitung über einen in dem Bereich der Nabe angeordneten Filter zu einem ebenfalls in dem Bereich der Nabe angeordneten Sammelbehälter geleitet.
Die EP-A-0 616 816 offenbart einen Einsatz zur Aufnahme von Blutbeuteln zum Einsatz in einer Zentrifuge zur Trennung von Blutbestandteilen mit einer Zwischenwand, die einen radial innen liegenden Blutbeutelbereich von einem radial aussen liegenden Produktbereich abteilt, wobei in dem Produktbereich eine Aufnahme für einen Filter vorgesehen ist, einem Produkt -Förderweg, der von dem Blutbeutelbereich über die Aufnahme für den Filter zu dem Produktbereich führt.

### Darstellung der Erfindung

### Technische Aufgabe

Es ist die Aufgabe der Erfindung, einen verbesserten Einsatz und eine Zentrifuge mit Einsatz zu schaffen, die eine verbesserte Ausbeute bei der Zellgewinnung und eine wirtschaftlichere Zellgewinnung ermöglichen.

### Technische Lösung

Die Aufgabe der Erfindung wird durch einen Einsatz nach Anspruch 1 und durch eine Zentrifuge nach Anspruch 10 gelöst. Vorteilhafte Ausführungsformen der Erfindung werden gemäß der abhängigen Ansprüche gelöst.

Ein erfindungsgemäßer Einsatz zur Aufnahme von Blutbeuteln, die zur Verwendung in einer Zentrifuge zur Trennung von Blutbestandteilen vorgesehen ist, weist eine Zwischenwand auf, die einen radial innen liegenden Blutbeutelbereich von einem radial außen liegenden Produktbereich abteilt. In dem Produktbereich ist eine Aufnahme für einen Filter vorgesehen. Ein Produkt-Förderweg führt von dem Blutbeutelbereich über die Aufnahme für den Filter zu dem Produktbereich. Der Produkt-Förderweg ist dabei derart definiert, dass er von dem Blutbeutelbereich kommend radial von außen und von unten in die Aufnahme für den Filter geführt ist.

Durch die erfindungsgemäße Definition des Produkt-Förderwegs und der Filterposition ist sichergestellt, dass rote Blutkörperchen, die versehentlich durch den Schlauch gefördert wurden, sich aufgrund der Fliehkraft an der Außenseite und Unterseite des Filters sammeln. Somit besteht keine Gefahr, dass die roten Blutkörperchen in den Produktbeutel weitergefördert werden.

Vorteilhaft kann die Aufnahme eine radial außerhalb der Zwischenwand liegende Außenwand aufweisen, die mit einer Führungseinrichtung zum Führen eines entlang des Produkt-Förderwegs geführten Schlauchs versehen ist. Die Führungseinrichtung kann dabei bevorzugt als Schlitz in der Außenwand der Aufnahme ausgeführt sein.

Vorteilhaft ist der Produkt-Förderweg von der Aufnahme für den Filter über eine oberhalb der Zwischenwand ausgebildete Aussparung weiter zu dem Produktbeutelbereich geführt.

Insbesondere kann der Blutbeutelbereich eine Abdeckung aufweisen, und der Produkt-Förderweg außerdem über Aussparungen in der Abdeckung definiert sein, wobei die Aussparungen zur Aufnahme eines Schlauchs und/oder einer Schlauchklemme vorgesehen sind.

Von der Aufnahme für den Filter kann der Produkt-Förderweg über in der Abdeckung ausgebildete zweite Aussparungen zu dem Produktbeutelbereich geführt sein. Dabei kann in einer der Aussparungen und/oder in einer der zweiten Aussparungen mindestens ein lichtempfindlicher Sensor vorgesehen sein. Insbesondere der zweite Sensor ermöglicht es, die Ausbeute bei der Zellgewinnung zu optimieren. Nach einem durch den ersten Sensor ausgegebenen "Achtung-" Signal kann die Fördergeschwindigkeit des Produktes reduziert werden um eine punktgenaue Messung am zweiten Sensor zu bewerkstelligen. Ausgehend von einer vorbestimmten Zusammensetzung des Produkts in dem Schlauch wird ein endgültiges Signal zum Beenden der Förderung, verbunden mit einem Signal die Schlauchklemmen zu schließen, ausgegeben.

Die zweiten Aussparungen können beispielsweise im Wesentlichen spiegelbildlich zu den ersten Aussparungen ausgebildet sein.

In der Abdeckung des Einsatzes können Betätigungseinrichtungen zur Betätigung einer Schlauchklemme bereitgestellt sein. Die Schlauchklemme ist dabei in einer der Aussparungen oder in einer der zweiten Aussparungen aufgenommen. Insbesondere können auch eine Schlauchklemme in einer der Aussparungen und eine Schlauchklemme in einer der zweiten Aussparungen aufgenommen sein. Die Betätigungseinrichtungen für die Schlauchklemmen ermöglichen ein zielgerichtetes und punktgenaues Beenden des Zellgewinnungsvorgangs und verhindern dabei, das Eintreten unerwünschter Blutbestandteile in den Produktbeutel.

Die Abdeckung des Einsatzes kann mit der Zwischenwand an einem ersten Punkt lösbar und an einem zweiten Punkt drehbar mit der Zwischenwand verbunden sein. Bei seitlich weggedrehter Abdeckung ist dann ein unterhalb der Abdeckung vorgesehener Blutbeutelbereich frei zugänglich ist. Die ermöglicht ein schnelles Austauschen des Blutbeutels in dem Blutbeutelbereich. Durch die drehbare Verbindung der Abdeckung mit der Zwischenwand kann der Blutbeutel und ein oder mehrere

Anschlussschläuche des Blutbeutels einfach positioniert werden. Dadurch sind die Schläuche und der Beutel binnen kurzer Zeit optimal fixiert.

Der Einsatz kann außerdem mit einer radial außen liegenden Auffangwanne vorgesehen sein, die den Produktbereich und teilweise den Blutbeutelbereich umfassen kann. Vorteilhaft ist in der Auffangwanne eine Greifeinrichtung vorgesehen, die das Handhaben der Auffangwanne und des von dieser teilweise umfassten Einsatzes erleichtert. als Greifeinrichtung können beispielsweise Fingerlöcher oder Handgriffe vorgesehen sein.

Der voranstehend beschriebene Einsatz ist zur Verwendung in einer Zentrifuge zur Trennung von Blutbestandteilen vorgesehen. Die Zentrifuge weist eine Nabe und einen um diese drehbaren Rotor auf. In dem Rotor können vorteilhaft die Nabe herum angeordnete Aufnahmekästen, die auch als Systemboxen bezeichnet werden, angeordnet sein, die zur Aufnahme der Einsätze dienen. Allerdings ist auch eine Zentrifuge möglich, in der lediglich ein Einsatz aufgenommen ist. Jeder Einsatz ist durch ein Betätigen eines mit dem Aufnahmekasten verbundenen Verriegelungselements frei aus dem Aufnahmekasten bzw. der Zentrifuge entnehmbar. Die Aufnahmekästen können dabei lösbar mit dem Rotor verbunden sein.

Dies ermöglicht ein schnelles Austauschen der Einsätze mit den getrennten Blutbestandteilen durch neue Einsätze mit noch nicht getrennten Blutbestandteilen und ermöglicht dabei eine höhere Produktionsausbeute bei der Zellgewinnung und eine optimierte Geräteauslastung.

Das Verriegelungselement kann in einem nicht betätigten Zustand eine Verriegelungsposition einnehmen. Dadurch wird ein sofortiges Verriegeln erreicht, sobald ein Einsatz in den Aufnahmekasten des Rotors eingesetzt wird. Das Verriegelungselement kann außerdem in dem Bereich der Nabe oder des Aufnahmekastens vorgesehen sein.

Außerdem kann das Verriegelungselement bei einem dem Einsatz zugeordneten Stützteil vorgesehen sein. Entsprechend ist dann der Einsatz zwischen dem Stützteil und einer Wand des Aufnahmekastens aufgenommen und lediglich in eine Richtung nach oben bewegbar. Diese Bewegung ist allerdings nur durch die Betätigung des Verriegelungselements möglich. Dadurch ist eine sichere Positionierung des Einsatzes in dem Aufnahmekasten für den Zentrifugiervorgang möglich.

Zum Halten des Einsatzes kann das Verriegelungselement in dem nicht betätigten Zustand mit einer der Zwischenwand gegenüberliegenden Seitenfläche der Abdeckung in Eingriff sein. Auf dem Verriegelungselement ist dabei ein Vorsprung ausgebildet, der eine Bewegung des Einsatzes nach oben verhindert.

Das Stützteil kann außerdem eine Kontaktstelle zur Herstellung einer elektrisch leitenden Verbindung zwischen dem Aufnahmekasten und dem Einsatz aufweisen. Die Kontaktstelle kann dabei aus einer Vielzahl von elektrischen Kontaktpunkten bestehen.

Bei dem Stützteil kann ein Druckelement vorgesehen sein, das radial in den Bereich unter der Abdeckung beweglich ist. Dies dient dazu, die getrennten Bestandteile aus dem Blutbeutel heraus in den Schlauch zu drücken, durch den sie weiter zu dem Filter und in den Produktbeutel geleitet werden.

Ein erster Abschnitt einer Leitung des Blutbeutels kann vorteilhaft nach oben und radial nach innen geführt sein. Dies verhindert ein unerwünschtes Austreten von Flüssigkeiten in den Schlauch, bevor die Blutbestandteile voneinander getrennt sind.

Der erfindungsgemäße Einsatz und die Zentrifuge sind einerseits für die Gewinnung von Zellen und Plasma aus Vollblut geeignet, sind aber auch für die Gewinnung von Zellen aus dem nach einem bekannten Zentrifugiervorgang verbliebenen "buffy coat" vorgesehen.

Dazu wird das "buffy coat" aus mehreren Blutbeuteln gemeinsam mit einer Additivlösung in einem neuen Blutbeutel gesammelt und vermischt. Der neue Blutbeutel entspricht dabei dem Blutbeutel gemäß der Erfindung. Der neue Blutbeutel kann vorteilhaft mit einem Schlauch und/oder einem insbesondere zum Filtern von Leukozyten vorgesehenen Filter vorgesehen sein.

### Kurze Beschreibung der Abbildungen der Zeichnungen

Im Folgenden wird ein Ausführungsbeispiel der Erfindung anhand der Figuren beschrieben. Dabei zeigen:

Fig. 1 eine Draufsicht des erfindungsgemäßen Einsatzes,

Fig. 2 eine perspektivische Ansicht des Einsatzes,

Fig. 3 eine entlang einer Symmetrielinie geschnittene, perspektivische Ansicht des Einsatzes,

Fig. 4 eine die Ansicht der Fig. 4 ergänzende geschnittene, perspektivische Ansicht des Einsatzes,

Fig. 5 eine weitere perspektivische und geschnittene Ansicht des Einsatzes,

Fig. 6 eine Unterseite einer Abdeckung des Einsatzes,

Fig. 7 eine perspektivische Ansicht eines Aufnahmekastens, und

Fig. 8a bis 8c schematische Schnittansichten des Einsatzes, aus denen die Zellgewinnung ersichtlich ist, und

Fig. 9 zeigt einen Strom des Blutprodukts durch den Filter.

### Weg(e) zur Ausführung der Erfindung

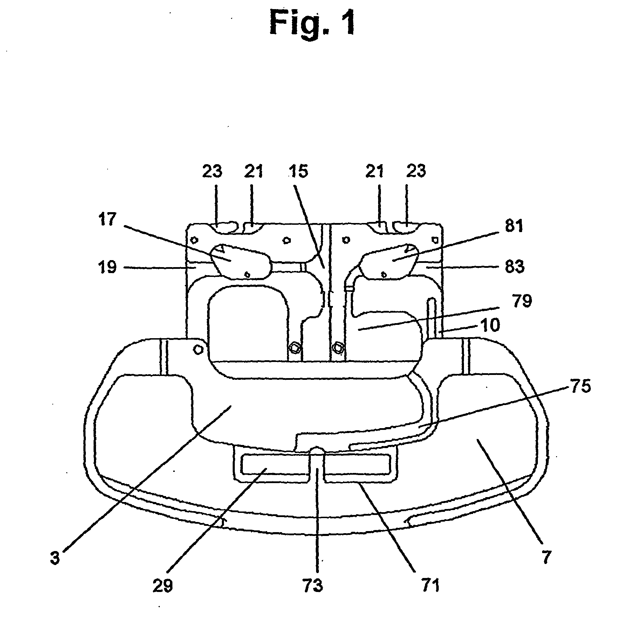

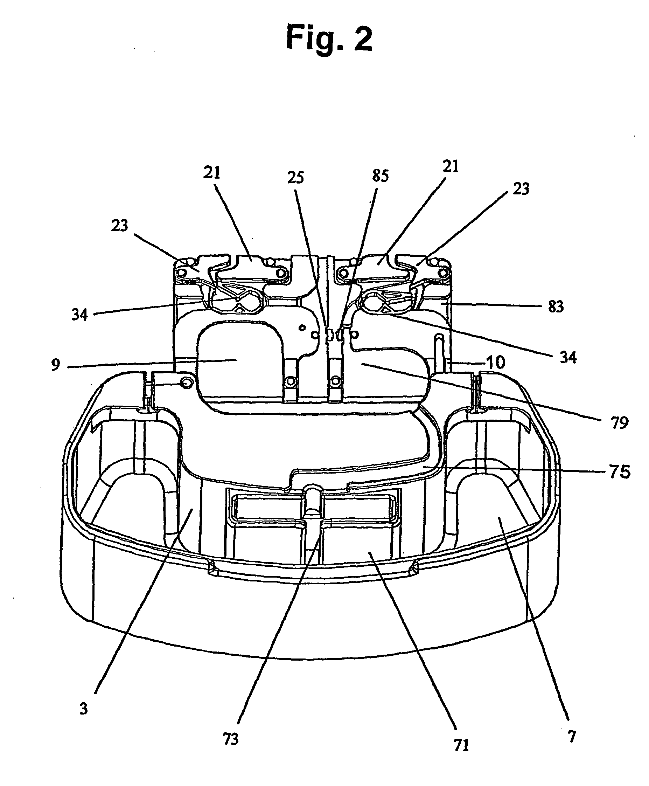

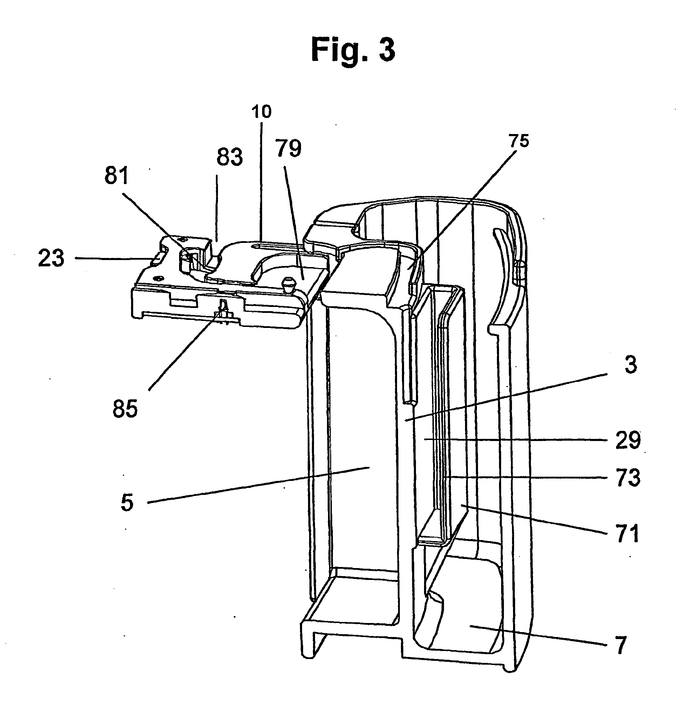

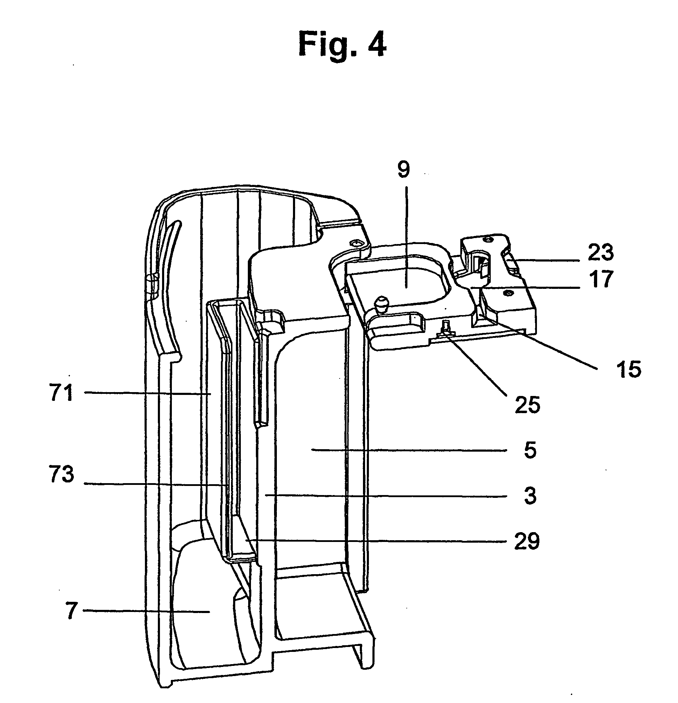

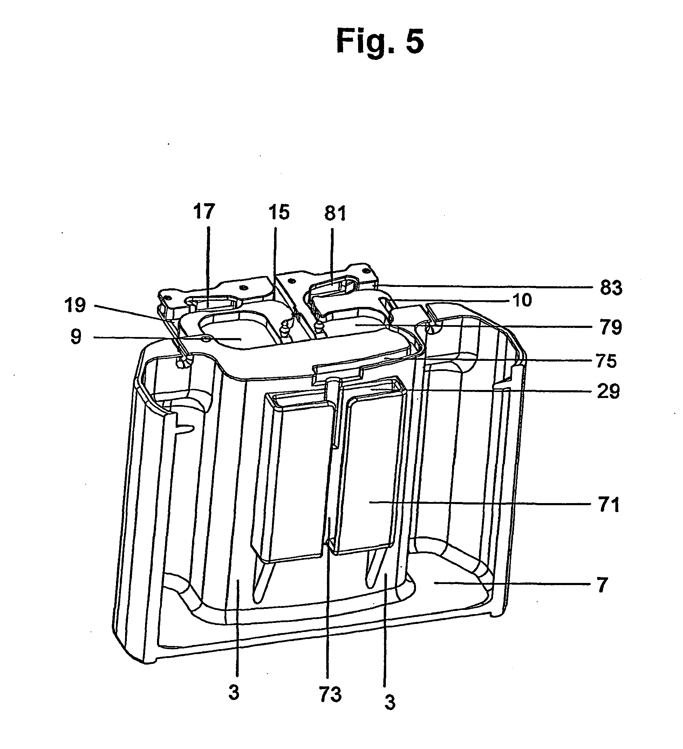

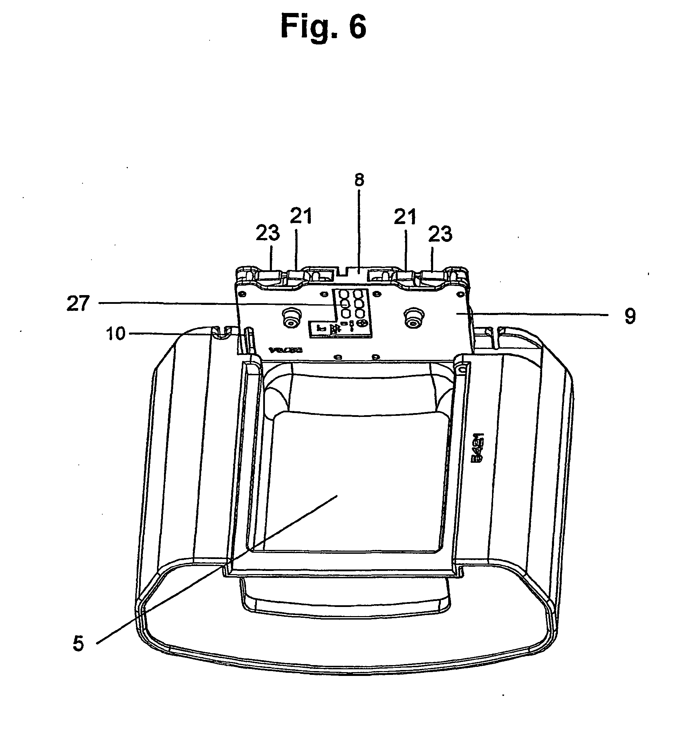

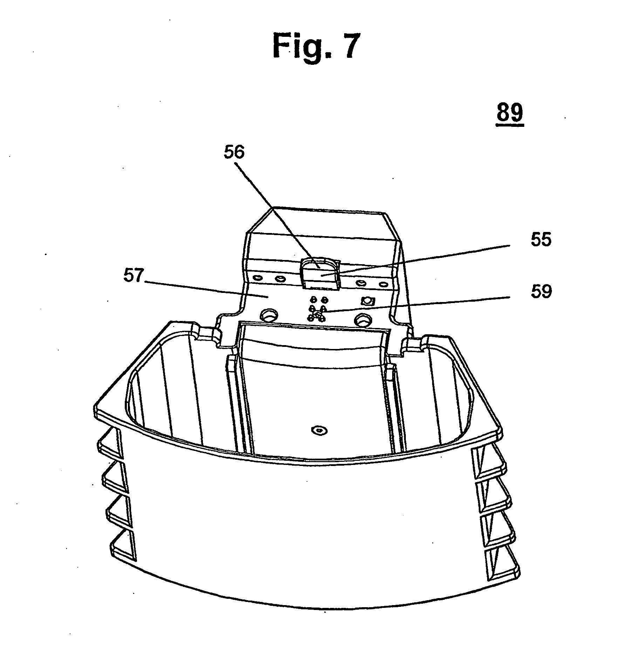

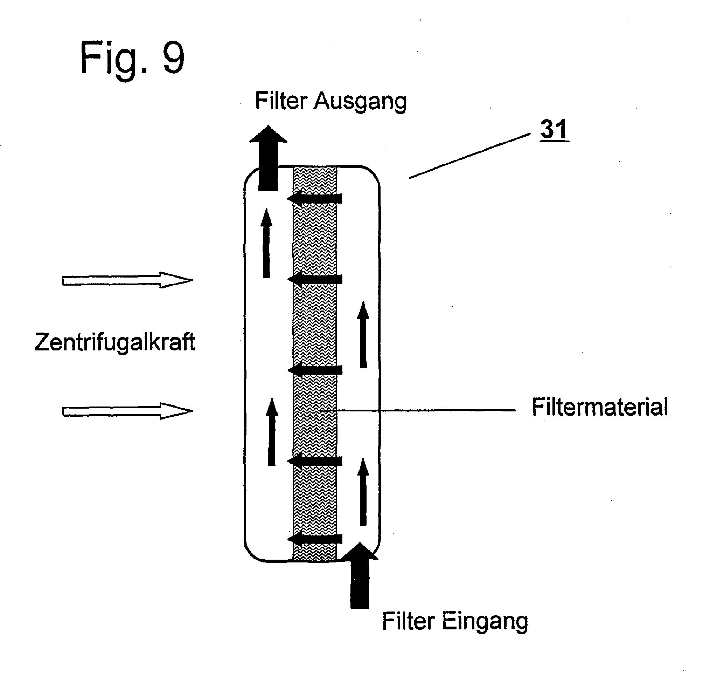

Ein Ausführungsbeispiel der Erfindung wird anhand der Figuren 1 bis 9 beschrieben.

Ein Einsatz 1 besteht im Wesentlichen aus einer Zwischenwand 3 und einer Abdeckung 9. Die Zwischenwand definiert einen Blutbeutelbereich 5 und einen Produktbeutelbereich 7. Ist der Einsatz 1 in eine Systembox 89 des Rotors einer Zentrifuge eingeschoben, liegt der Blutbeutelbereich 5 radial innerhalb von der Zwischenwand 3, während der Produktbeutelbereich 7 radial außerhalb von der Zwischenwand 3 liegt. Als Systembox 89 wird dabei ein Aufnahmekasten 89 gemäß der Erfindung bezeichnet.

Über dem Blutbeutelbereich 5 ist eine Abdeckung 9 vorgesehen. Diese ist im Wesentlichen rechteckig ausgebildet und in einem geschlossenen Zustand mit einer Längsseite mit der Zwischenwand 3 in Berührung. An einem Eckpunkt ist die Abdeckung drehbar in der Zwischenwand gelagert, während sie an einem zweiten Eckpunkt mittels einem Riegel 10 mit der Zwischenwand 3 in Eingriff gerät. Zum Öffnen der Abdeckung wird auf den Riegel 10 ein Druck ausgeübt, und danach die Abdeckung zur Seite gedreht. Dadurch ist der Blutbeutelbereich 5 frei zugänglich und kann mit einem Blutbeutel 35 befüllt werden.

Durch den einfachen Drehmechanismus kann ein Schlauch 36 und ein Blutbeutel 35 beim Schließen der Abdeckung 9 einfach und mit einem geringen Aufwand in einer vorgesehenen Lage gehalten und durch Schließen der Abdeckung 9 in dieser vorgesehenen Lage fixiert werden.

Nach dem Schließen der Abdeckung 9 ist es möglich, den Schlauch 36 in Aussparungen 15, 19 einzulegen, die an der Oberseite der Abdeckung 9 ausgebildet sind. Ein erster lichtempfindlicher Sensor 25 ist in der Aussparung 15 vorgesehen.

Eine mit dem Blutbeutel mitgelieferte, auf dem Schlauch vorhandene Schlauchklemme 34 in geschlossenem Zustand, beispielsweise eine von der Firma "Halkey Roberts" hergestellte, wird in einer ebenfalls an der Oberseite der Abdeckung 9 ausgebildeten Aussparung 17 aufgenommen.

Das von dem Blutbeutel 35 aus gesehene ferne Ende des Schlauchs 36 wird zu dem Produktbeutelbereich 7 geführt und ist dort mit einem Leukozytenfilter 31 verbunden, der in einer Aufnahme 29 aufgenommen ist. Dabei ist der Schlauch 36 radial von außen und von unten in den Leukozytenfilter 31 eingeführt. Das Einlegen von Filter 31 und Schlauch 36 wird durch einen Schlitz 73 in einer Außenwand 71 der Aufnahme 29 ermöglicht. Durch den Schlitz 73 kann der mit dem Filter 31 verbundene Schlauch 36 von oben nach unten verschoben werden, wenn der Filter in die Aufnahme eingebracht wird, sodass der Schlauch radial von außen und von unten zu dem Filter geführt ist.

Nach dem Filter 31 wird der Schlauch 36 über zweite in der Abdeckung 9 vorgesehene Aussparungen 75, 79, 81, 83, die im Wesentlichen spiegelbildlich zu den Aussparungen 15, 17, 19 angeordnet sind, zu dem Produktbeutel 33 geführt, der sich radial außerhalb von der Aufnahme 29 befindet.

In der Aussparung 81 ist eine zweite Schlauchklemme 34 vorgesehen. Ein zweiter lichtempfindlicher Sensor 85 befindet sich in der Aussparung 79. Zum Betätigen der Klemmen 34 sind innerhalb der Abdeckung 9 jeweils zwei Stangen 21, 23 als Betätigungseinrichtung so durch die Abdeckung geführt, dass eines ihrer Enden geringfügig aus einer der Zwischenwand 3 gegenüberliegenden Seitenfläche 8 der Abdeckung 9 herausragt und das andere Ende sich in dem Bereich der die Klemme 34 aufnehmenden Vertiefung 17 befindet. Durch das Ausüben eines Drucks auf eines der aus der Seitenfläche 8 ragenden Enden kann somit die handelsübliche Klemme 34 geöffnet bzw. geschlossen werden. Gemäß der Ausführungsform sind die Schlauchklemmen 34 sowohl einzeln als auch pneumatisch betätigbar.

Nach der Bestückung des Einsatzes 1 kann der Einsatz 1 in die Systembox 89 des Rotors einer Zentrifuge eingesetzt werden. Dabei ruht die der Zwischenwand 3 gegenüberliegende Seitenfläche 8 der Abdeckung 9 an einem Stützteil 57 der Systembox 89, das in dem Bereich einer Nabe der Zentrifuge vorgesehen ist. Bei dem Stützteil 57 befindet sich außerdem ein stabförmiges Verriegelungselement 55, das an seiner radialen Außenseite einen Vorsprung 56 aufweist. Durch das Einsetzen des Einsatzes 1 gleitet die Seitenfläche 8 der Abdeckung 9 über den Vorsprung 56 und bewegt das Verriegelungselement 55 radial nach innen, bis die Seitenfläche 8 unter den Vorsprung 56 gerät, und das Verriegelungselement 55 in die ursprüngliche Lage zurückfedert, und somit eine Verschiebung des Einsatzes 1 nach oben verhindert. Der Einsatz 1 ist nun fest zwischen der Außenwand der Systembox 89 und dem Stützelement positioniert.

Gemäß dem Ausführungsbeispiel ist der Rotor der Zentrifuge für sechs Systemboxen 89 mit jeweils einem Einsatz 1 ausgelegt. Nach dem Einschieben aller Einsätze 1 wird die Zentrifuge gestartet. Durch die Fliehkraft kommt es zu der gewünschten Trennung der Blutbestandteile. Da sich das durch eine Additivlösung verdünnte "buffy coat" in dem Blutbeutel 35 befindet, werden dessen leichtere Bestandteile radial innen verbleiben, während schwerere Bestandteile d.h. die roten Blutkörperchen sich außen sammeln.

Um den gewünschten Blutbestandteil - gemäß der Ausführungsform sind dies die Blutplättchen - in hoher Qualität d.h. ohne Beimengung anderer Blutzellen aus dem Blutbeutel zu fördern, wird nach der Auftrennung der Bestandteile mittels einem bekannten Druckkissen 61 ein leichter Druck auf den Blutbeutel ausgeübt, sodass nach dem Öffnen der Klemmen 34 die plättchenreiche Lösung beginnt, in den nach oben und radial nach innen geführten Schlauch 36 aufzusteigen. Durch den Schlauch 36 wird die plättchenreiche Lösung in den Leukozytenfilter 31 geleitet, in den sie radial von außen und unten eintritt.

In dem Leukozytenfilter 31 werden die unerwünschten Leukozyten, das sind die weißen Blutkörperchen, entfernt. Aufgrund der erfindungsgemäßen Anordnung des Schlauchs 36 mit dem Filter 31 findet die Filtration entgegen der Zentrifugalkraft bzw. Fliehkraft statt. Dadurch werden schwerere Blutbestandteile wie ungewollt mitgeförderte rote Blutkörperchen in einer radial außen liegenden Filtereingangskammer abgefangen.

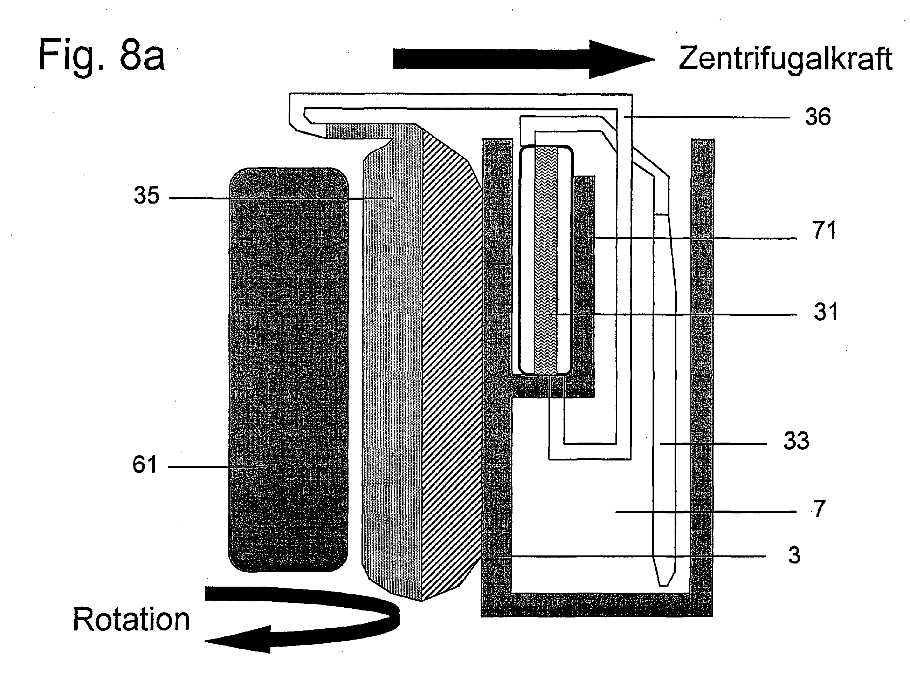

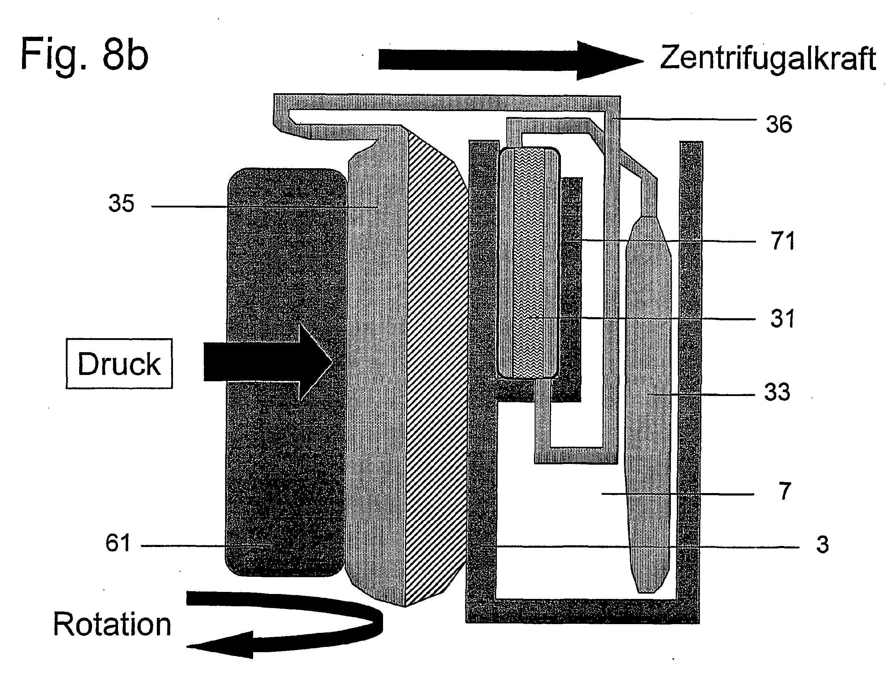

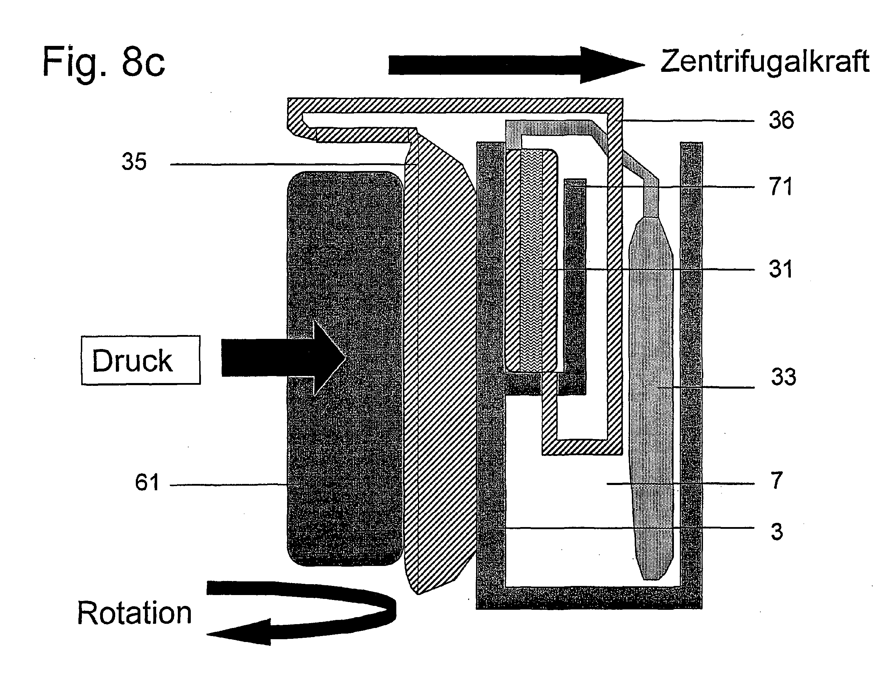

Nach dem Passieren des Leukozytenfilters 31 fließt die plättchenreiche Lösung schließlich weiter durch den Schlauch 36 in einen Produktbeutel 33, in dem sie gesammelt wird. Der Produktbeutel 33 ist vorzugsweise bereits als endgültiger Lagerbeutel für das Produkt ausgebildet. Der gesamte Vorgang ist in Fig. 8a bis 8c schematisch dargestellt.

Um eventuell vorhandene Luft in dem Filter zu entfernen, wird am Beginn der Produktüberleitung für eine bestimmte Volumenmenge die Fließgeschwindigkeit niedrig gehalten, und dadurch ermöglicht, dass sich der Filter zuverlässig und vollständig mit dem Blutprodukt füllt. Nach der Überleitung dieser bestimmten Volumenmenge wird mittels einer geeigneten Steuerung des Druckkissens, die Fördergeschwindigkeit für eine bestimmte zweite Volumenmenge erhöht. Während dieses zweite Volumen gefördert wird, besteht nur sehr geringe Gefahr, dass rote Blutkörperchen das Blutprodukt (hier das Thrombozytenkonzentrat) verunreinigen. Falls dies doch passiert, wird diese geringe Anzahl von roten Blutkörperchen, wegen der Führung des Schlauchs 36 von radial außen und unten in den Filter und der Wirkung der Zentrifugalkraft bzw. Fliehkraft, in dem unteren und äußeren Bereich des Filters gesammelt.

Nach dem Überleiten des zweiten Volumens wird der erste lichtempfindliche Sensor aktiviert und die Strömungsgeschwindigkeit des Blutprodukts in dem Schlauch 36 reduziert.

Erfasst der erste lichtempfindliche Sensor 25 einen vorbestimmten Anteil roter Blutkörperchen in der thrombozytenreichen Lösung, gibt er ein Signal ab, durch das die Strömungsgeschwindigkeit abermals reduziert wird. Außerdem wird der hinter dem Filter 31 angeordnete zweite lichtempfindliche Sensor 85 aktiviert.

In dieser Phase kann auch eine größere Anzahl roter Blutkörperchen in den Filter 31 eindringen und diesen sogar passieren, bis der zweite lichtempfindliche Sensor 85 einen vorbestimmten Anteil roter Blutkörperchen in dem Blutprodukt erfasst und ein Signal zum Beenden des Zellgewinnungsvorgangs ausgibt. Durch dieses Signal werden die Schlauchklemmen 34 durch das Betätigen der Stange 23 geschlossen, sodass die roten Blutkörperchen in dem Filter zuverlässig von dem Thrombozytenkonzentrat im Produktbeutel getrennt sind. Die Betätigung der Stange erfolgt über einen in der Systembox 89 vorgesehenen Stellmechanismus.

Alternativ zur Beendigung durch den zweiten lichtempfindlichen Sensor 85, kann der Zellgewinnungsvorgang auch nach dem Verstreichen eines bestimmten Zeitraums nach dem Aktivieren des zweiten lichtempfindlichen Sensors 85 beendet werden.

In der Ausführungsform sind insgesamt sechs Einsätze in der Zentrifuge vorgesehen. Durch die vorangehend beschriebene Steuerung des Zellgewinnungsvorgangs in einem Einsatz 1 mittels Druckkissen 61 dem Öffnen und Schließen der Schlauchklemmen 34, und der Prozeßkontrolle mittels der zwei lichtempfindlichen Sensoren 25, 85 ist es möglich, die Zellgewinnung in den Einsätzen der übrigen Systemboxen 89 fortzusetzen da die beschriebene Prozeßsteuerung individuell für jede Kombination aus Einsatz und Systembox stattfindet.

Zur Übertragung der Steuer- und anderer elektrischer Signale ist auf dem Stützteil 57 der Systembox 89 eine elektrische Kontaktstelle in Form von einzelnen Kontaktpunkten 59 vorgesehen. An der Unterseite der Abdeckung 9 befinden sich den Kontaktpunkten 59 zugeordnete Kontaktflächen 27, die bei dem Einschieben des Einsatzes 1 in die Systembox mit den Kontaktpunkten 59 in Berührung geraten. Die Kontaktpunkte 59 sind zu diesem Zweck federnd gelagert.

Zur leichteren Handhabung einerseits und für den Fall, bei dem Blutbestandteile aufgrund von einer Beschädigung der Beutel 33, 35, des Schlauchs 36 oder des Filters 31 austreten, ist der Einsatz 1 von einer radial inneren Richtung in eine Auffangwanne eingeführt. In dem Fall einer Beschädigung wird der ausgetretene Blutbestandteil zum Großteil in der Auffangwanne gesammelt, sodass es nur zu geringfügigen Verschmutzungen der Systembox 89 oder des Rotors selbst kommt. Die Systembox 89 kann in einem derartigen Fall einfach aus dem Rotor ausgebaut werden.

Nach der Beendigung der Zellgewinnung wird jeder der Einsätze 1 entnommen, indem auf das Verriegelungselement 55 ein leichter Druck ausgeübt wird, um dieses radial nach innen zu bewegen.

Gleichzeitig werden die Einsätze 1 an Fingerlöchern der Auffangwanne erfasst und nach oben aus der Systembox 89 der Zentrifuge herausgehoben, um sofort durch neue, frisch bestückte Einsätze 1 ersetzt zu werden. Während der folgenden Zellgewinnung können die Blutbeutel 35 und Produktbeutel 33 aus den ausgetauschten Einsätzen 1 entnommen werden und diese wieder neu bestückt werden.

Ein Einsatz (1) zur Aufnahme von Blutbeuteln (35) zum Einsatz in einer Zentrifuge dient zur Trennung von Blutbestandteilen. Der Einsatz (1) hat eine Zwischenwand (3), die einen radial innen liegenden Blutbeutelbereich (5) von einem radial außen liegenden Produktbereich (7) abteilt, wobei in dem Produktbereich (7) eine Aufnahme (29) für einen Filter (31) vorgesehen ist, einem Produkt-Förderweg (36), der von dem Blutbeutelbereich (5) über die Aufnahme (29) für den Filter (31) zu dem Produktbereich (7) führt. Der Produkt-Förderweg (36) führt von dem Blutbeutelbereich (5) kommend radial von außen und von unten in die Aufnahme (29) für den Filter (31).

## Patentansprüche

1. Einsatz (1) zur Aufnahme von Blutbeuteln (35) zum Einsatz in einer Zentrifuge zur Trennung von Blutbestandteilen mit:
einer Zwischenwand (3), die einen radial innen liegenden Blutbeutelbereich (5) von einem radial aussen liegenden Produktbereich (7) abteilt, wobei in dem Produktbereich (7) eine Aufnahme (29) für einen Filter (31) vorgesehen ist,
einem Produkt-Förderweg (36), der von dem Blutbeutelbereich (5) über die Aufnahme (29) für den Filter (31) zu dem Produktbereich (7) führt,
**dadurch gekennzeichnet, dass**
der Produkt-Förderweg (36) von dem Blutbeutelbereich (5) kommend radial von aussen und von unten in die Aufnahme (29) für den Filter (31) führt.

2. Einsatz (1) nach Anspruch 1, wobei die Aufnahme (29) eine radial ausserhalb der Zwischenwand (3) liegende Aussenwand (71) aufweist, die mit einer Führungseinrichtung (73) zum Führen eines entlang des Produkt-Förderwegs geführten Schlauchs (36) versehen ist.

3. Einsatz (1) nach Anspruch 1 bis 2, wobei der Produkt-Förderweg von der Aufnahme (29) für den Filter (31) weiter über eine oberhalb der Zwischenwand (3) ausgebildete Aussparung (75) zu dem Produktbeutelbereich (7) geführt ist.

4. Einsatz (1) nach Anspruch 1 bis 3, wobei der Blutbeutelbereich (5) eine Abdeckung (9) aufweist, und der Produkt-Förderweg ausserdem über Aussparungen (15, 17, 19) in der Abdeckung (9) definiert ist, wobei die Aussparungen (15, 17, 19) zur Aufnahme eines Schlauchs (36) und/oder einer Schlauchklemme (34) vorgesehen sind.

5. Einsatz (1) nach Anspruch 4, wobei der Produkt-Förderweg von der Aufnahme (29) für den Filter über in der Abdeckung (9) ausgebildete zweite Aussparungen (79, 81 , 83) zu dem Produktbeutelbereich (7) geführt ist.

6. Einsatz (1) nach Anspruch 4 und 5, wobei in einer der Aussparungen (15, 17, 19) und/oder in einer der zweiten Aussparungen (79, 81, 83) mindestens ein lichtempfindlicher Sensor vorgesehen ist.

7. Einsatz (1) nach Anspruch 4 bis 6, wobei in der Abdeckung (9) Betätigungseinrichtungen (21, 23) zur Betätigung einer in einer der Aussparungen (15, 17, 19) und/oder der zweiten Aussparungen (79, 81 , 83) aufgenommenen Schlauchklemme (34) bereitgestellt sind.

8. Einsatz nach einem der Ansprüche 4 bis 7*,* wobei die Abdeckung (9) mit der Zwischenwand an einem ersten Punkt lösbar und an einem zweiten Punkt drehbar mit der Zwischenwand (3) verbunden ist und bei seitlich weggedrehter Abdeckung (9) ein unterhalb der Abdeckung (9) vorgesehener Blutbeutelbereich frei zugänglich ist.

9. Einsatz nach einem der Ansprüche 1 bis 8, wobei eine radial aussen liegende Auffangwanne vorgesehen ist, die den Produktbereich (7) und teilweise den Blutbeutelbereich (5) umfass
wobei die Auffangwanne mit einer Greifeinrichtung zur Handhabung derselben und des Einsatzes versehen ein kann.

10. Zentrifuge zur Trennung von Blutbestandteilen mit:
einem um eine Nabe (51) drehbaren Rotor, mindestens einem in dem Rotor aufgenommenen und teilweise um die Nabe herum angeordneten Einsatz (1) nach einem der Ansprüche 1 bis 12, zur Aufnahme von Blutbeuteln (35), **gekennzeichnet durch** ein mit dem Rotor verbundenes Verriegelungselement (55), das dem Einsatz (1) zugeordnet ist, und bei dessen Betätigung der Einsatz (1) frei aus dem Rotor entnehmbar ist.

11. Zentrifuge nach Anspruch 10, wobei in dem Rotor mindestens ein Aufnahmekasten (89) zur Aufnahme eines Einsatzes (1) vorgesehen ist
der bevorzugt lösbar mit dem Rotor verbunden ist und das Verriegelungselement (55) bevorzugt an dem Aufnahmekasten vorgesehen ist.

12. Zentrifuge nach einem der Ansprüche 10 bis 11, wobei der Einsatz (1) in einer radial aussen liegenden Auffangwanne aufgenommen ist und zusammen mit dieser in den Rotor einsetzund entnehmbar sind.

13. Zentrifuge nach einem der Ansprüche 10 bis 12, wobei das Verriegelungselement (55) bei der Nabe (51) des Rotors angeordnet ist, und/oder wobei das Verriegelungselement (55) bei einem dem Einsatz (1) zugeordneten Stützteil (57) vorgesehen ist.

14. Zentrifuge nach einem der Ansprüche 10 bis 13, wobei das Verriegelungselement (55) in dem nicht betätigten Zustand mit einer der Zwischenwand (3) gegenüberliegenden Seitenfläche (8) der Abdeckung (9) in Eingriff ist und ein Vorsprung (56) auf dem Verriegelungselement (55) eine Bewegung des Einsatzes (1) nach oben verhindert.

15. Zentrifuge nach einem der Ansprüche 13 oder 14, wobei das Stützteil (57) und/oder der Aufnahmekasten (89) eine Kontaktstelle (59) zur Herstellung einer elektrisch leitenden Verbindung zwischen dem Rotor und dem Einsatz (1) aufweist.

16. Zentrifuge nach einem der Ansprüche 10 bis 15, wobei in dem Bereich der Nabe (51) des Rotors ein Stellmechanismus zur Betätigung von in der Einsatz vorgesehenen Betätigungseinrichtungen (21, 23) vorgesehen ist.

17. Zentrifuge nach Anspruch 16, wobei die Betätigung durch den Stellmechanismus pneumatisch oder elektrisch oder hydraulisch erfolgt.

18. Zentrifuge nach einem der Ansprüche 10 bis 17, wobei in dem Bereich der Nabe (51) ein radial nach aussen verschiebbares Druckelement (61) zum Ausüben eines Drucks auf einen Blutbeutel (35) vorgesehen ist.

## Claims

1. A cartridge (1) for accommodating blood bags (35), to be inserted into a centrifuge for the separation of blood components, comprising:
a partition wall (3) which separates a blood bag section (5) positioned radially inside from a product section (7) positioned radially outside, wherein a fixture (29) for a filter (31) is provided in the product section (7),
a product transport path (36) which leads from the blood bag section (5) via the fixture (29) for the filter (31) to the product section (7),
**characterized in that**
the product transport path (36) coming from the blood bag section (5) leads radially from the outside and from below into the fixture (29) for the filter (31).

2. A cartridge (1) according to claim 1, wherein the fixture (29) comprises an outer wall (71) positioned radially outside the partition wall (3) and having a guiding means (73) for guiding a tube (36) leading along the product transport path.

3. A cartridge (1) according to claims 1 to 2, wherein the product transport path leads from the fixture (29) for the filter (31) further via a recess (75) provided above the partition wall (3) to the product bag section (7).

4. A cartridge (1) according to claims 1 to 3, wherein the blood bag section (5) comprises a cover (9), and the product transport path is furthermore defined by recesses (15, 17, 19) in the cover (9), wherein the recesses (15, 17, 19) are provided for holding a tube (36) and/or a tube clamp (34).

5. A cartridge (1) according to claim 4, wherein the product transport path leads from the fixture (29) for the filter via second recesses (79, 81, 83) provided in the cover (9) to the product bag section (7).

6. A cartridge (1) according to claims 4 and 5, wherein at least one photo sensor is provided in one of the recesses (15, 17, 19) and/or in one of the second recesses (79, 81, 83).

7. A cartridge (1) according to claims 4 to 6, wherein operating means (21, 23) for operating a tube clamp (34) held in one of the recesses (15, 17, 19) and/or the second recesses (79, 81, 83) are provided in the cover (9).

8. A cartridge according to one of claims 4 to 7, wherein the cover (9) is detachably connected to the partition wall (3) at a first point, and is pivotally connected to the partition wall (3) at a second point, and a blood bag section (5) provided below the cover (9) is freely accessible when the cover (9) is laterally pivoted out of the way.

9. A cartridge according to one of claims 1 to 8, wherein a collecting tank positioned radially outside is provided and embraces the product section (7) and parts of the blood bag section (5), wherein the collecting tank can be provided with a handling means for handling the same and the cartridge.

10. The centrifuge for the separation of blood components, comprising:
a rotor revolving about a hub (51),
at least one cartridge (1) for accommodating blood bags (35) according to one of claims 1 to 12, held in the rotor and partly arranged around the hub,
**characterized by**
a locking element (55) connected to the rotor and assigned to the cartridge (1), upon the operation of which the cartridge (1) can be removed freely from the rotor.

11. A centrifuge according to claim 10, wherein at least one accommodating box (89) for accommodating a cartridge (1) is provided in the rotor, which preferably is detachably connected to the rotor and the locking element (55) preferably is provided at the accommodating box.

12. A centrifuge according to one of claims 10 to 11, wherein the cartridge (1) is accommodated in a collecting tank positioned radially outside and, together therewith, can be inserted into and removed from the rotor.

13. A centrifuge according to one of claims 10 to 12, wherein the locking element (55) is positioned at the hub (51) of the rotor and/or wherein the locking element (55) is provided at a support (57) assigned to the cartridge (1).

14. A centrifuge according to one of claims 10 to 13, wherein the locking element (55) in its non-operated state is engaged with a side surface (8) of the cover (9), located opposite the partition wall (3), and a projection (56) on the locking element (55) prevents an upward movement of the cartridge (1).

15. A centrifuge according to claim 13 or 14, wherein the support (57) and/or the accommodating box (89) comprise(s) a contact pad (59) for establishing an electrically conductive connection between the rotor and the cartridge (1).

16. A centrifuge according to one of claims 10 to 15, wherein, in the area of the hub (51) of the rotor, an actuating mechanism is provided for operating the operating means (21, 23) provided in the cartridge.

17. A centrifuge according to claim 16, wherein the operation of the actuating mechanism is effected pneumatically or electrically or hydraulically.

18. A centrifuge according to one of claims 10 to 17, wherein, in the area of the hub (51), a pressing element (61) which can be displaced radially outwards is provided for applying pressure onto a blood bag (35).

## Revendications

1. Insert (1), pour recevoir des poches à sang (35), pour utilisation dans une centrifugeuse pour la séparation des composants sanguins, avec ;
une paroi intermédiaire (3), subdivisant une zone à poche à sang (5), située radialement intérieurement, vis-à-vis d'une zone à produit (7) située radialement extérieurement, un logement (29) pour un filtre (31) étant prévu dans la zone à produit (7),
une voie de transfert de produit (36), allant de la zone à poche à sang (5) à la zone à produit (7), en passant par le logement (29) pour le filtre (31),
**caractérisé en ce que**
la voie de transfert de produit (36) chemine, en venant de la zone à poche à sang (5), radialement de l'extérieur et du bas, pour aller dans le logement (29) pour le filtre (31).

2. Insert (1) selon la revendication 1, **caractérisé en ce que** le logement (29) présente une paroi extérieure (71), située à l'extérieur de la paroi intermédiaire (3), munie d'un dispositif de guidage (73), pour guider un tuyau (36) guidé le long de la voie de transfert de produit (36).

3. Insert (1) selon les revendications 1 à 2, **caractérisé en ce que** la voie de transfert de produit est guidée, du logement (29) pour le filtre (31), en continuant par un évidemment (75) réalisé au-dessus de la paroi intermédiaire (3), vers la zone à poche à produit (7).

4. Insert (1) selon les revendications 1 à 3, **caractérisé en ce que** la zone à poche à sang (5) présente un couvercle (9), et la voie de transfert de produit est en outre définie par des évidements (15, 17, 19) réalisés dans le couvercle (9), les évidements (15, 17, 19) étant prévus pour recevoir un tuyau (36) et/ou un collier de serrage à tuyau (34).

5. Insert (1) selon la revendication 4, **caractérisé en ce que** la voie de transfert de produit est guidée, du logement (29) pour le filtre, en passant par des deuxièmes évidements (79, 81, 83) réalisés dans le couvercle (9), vers la zone à poche à produit (7).

6. Insert (1) selon les revendications 4 et 5, **caractérisé en ce qu'**au moins un capteur photosensible est prévu dans l'un des évidements (15, 17, 19) et/ou dans l'un des deuxièmes évidements (79, 81, 83).

7. Insert (1) selon les revendications 4 à 6, **caractérisé en ce que** des dispositifs d'actionnement (21, 23), pour l'actionnement d'un collier de serrage à tuyau (34), logé dans l'un des évidements (15, 17, 19) et/ou des deuxièmes évidements (79, 81, 83), sont fournis dans le couvercle (9) .

8. Insert (1) selon l'une des revendications 4 à 76, **caractérisé en ce que** le couvercle (9) est relié de manière désolidarisable, en un premier point, à la paroi intermédiaire et relié, avec possibilité de rotation, à la paroi intermédiaire (3) et une zone à poche à sang, prévue au-dessous du couvercle (9), est librement accessible lorsque le couvercle (9) est écarté latéralement par une rotation.

9. Insert (1) selon les revendications 1 à 8, **caractérisé en ce qu'**est prévue une auge de captage située radialement
extérieurement, enveloppant la zone à produit (7) et, partiellement, la zone à poche à sang (5).

10. Centrifugeuse, pour la séparation de composants sanguins, avec :
un rotor, susceptible de tourner autour d'un moyeu (51),
au moins un insert (1) selon l'une des revendications 1 à 12, logé dans le rotor et disposé partiellement autour du moyeu, pour recevoir des poches à sang (35), **caractérisé par** un élément de verrouillage (55), relié au rotor, associé à l'insert (1) et lors de l'actionnement duquel l'insert (1) est susceptible d'être retiré librement du rotor.

11. Centrifugeuse selon la revendication 10, **caractérisée en ce qu'**au moins une boîte de logement (89), pour recevoir un insert (1), est prévue dans le rotor,
est reliée de manière désolidarisable au rotor et l'élément de verrouillage (55) est, de préférence, prévu sur la boîte de logement.

12. Centrifugeuse selon l'une des revendications 10 à 11, **caractérisée en ce que** l'insert (1) est logé dans une auge de captage, située radialement extérieurement, et, conjointement avec celle-ci, est susceptible d'être inséré dans le rotor et d'en être retiré.

13. Centrifugeuse selon l'une des revendications 10 à 12, **caractérisée en ce que** l'élément de verrouillage (55) est disposé au moyeu (51) du rotor et/ou
l'élément de verrouillage (55) est prévu à une partie d'appui (57) associée à l'insert (1).

14. Centrifugeuse selon l'une des revendications 10 à 13, **caractérisée en ce que**, à l'état non actionné, l'élément de verrouillage (55) est en prise avec une face latérale (8), opposée à la paroi intermédiaire (3), du couvercle (9), et une saillie (56), disposée sur l'élément de verrouillage (55), empêche tout déplacement vers le haut de l'insert (1).

15. Centrifugeuse selon l'une des revendications 13 ou 14, **caractérisée en ce que** la partie d'appui (57) et/ou la boîte de logement (89) présente(nt) un emplacement de contact (59) pour établir une liaison conductrice de l'électricité entre le rotor et l'insert (1).

16. Centrifugeuse selon l'une des revendications 10 à 15, **caractérisée en ce qu'**un mécanisme de manoeuvre, servant à l'actionnement de dispositifs d'actionnement (21, 23) prévus dans l'insert, est prévu dans la zone du moyeu (51) du rotor.

17. Centrifugeuse selon la revendication 160, **caractérisée en ce que** l'actionnement s'effectue, pneumatiquement, ou électriquement ou hydrauliquement, au moyen du mécanisme de manoeuvre.

18. Centrifugeuse selon l'une des revendications 10 à 17, où un élément de pressage (61), déplaçable radialement vers l'extérieur, pour exercer une pression sur une poche à sang (35), est prévu dans la zone du moyeu (51).
